# EUROPEAN PATENT SPECIFICATION

(11) **EP 4 450 021 B1**
(45) Date of publication and mention of the grant of the patent: **24.06.2026**
(21) Application number: 24170941.9
(22) Date of filing: 18.04.2024
(51) Int. Cl.: A61C 8/00, A61B 17/86, A61B 17/88, B25B 15/00, B25B 23/00

(54) **SCREWDRIVER FOR DENTAL IMPLANTOLOGY**
SCHRAUBENDREHER FÜR DIE ZAHNIMPLANTOLOGIE
TOURNEVIS D'IMPLANTOLOGIE DENTAIRE

(30) Priority: 21.04.2023 IT 202300007974
(43) Date of publication of application: 23.10.2024
(73) Proprietor: Sweden & Martina SpA, 35020 Due Carrare (PD) (IT)
(72) Inventor: MARTINA, Alberto, 35020 DUE CARRARE (PD) (IT)
(74) Representative: Rocchetto, Elena

(56) References cited:
- US-A1- 2014 186 797
- US-A1- 2016 151 127
- US-A1- 2019 274 793
- US-A1- 2022 313 400
- CUSANO C ET AL: "THE SLICING TECHNIQUE FOR THE EVALUATION OF THE FORMAL EFFICIENCY: A COMPARATIVE STUDY. (AIMETA 2017)", AIMETA 2017, XXIII CONFERENCE, THE ITALIAN ASSOCIATION OF THEORETICAL AND APPLIED MECHANICS, 7 September 2017 (2017-09-07), pages 1 - 12, XP093371472

## Description

### TECHNICAL FIELD

The present patent relates to screwdrivers for dental implantology and in particular it concerns a new screwdriver with a specially configured tip suitable for screwing in prosthetic screws even in the presence of angled holes. The present patent also concerns the screw suited to be used with the screwdriver and a joining element suited to be used with the screwdriver to tighten a screw.

### BACKGROUND

Prosthetic screws are known which are intended to be screwed in dental implants to constrain the post and cores to the dental implants themselves.

Prosthetic screws for angled holes are also known, that is, prosthetic screws specially configured to be screwed in dental implants in the case where the screwdriver cannot be inserted in a coaxial position with respect to the screw itself.

In such cases, the screw head is shaped in such a way as to allow it to be coupled with the screwdriver without being coaxial with the latter, which is therefore positioned with its axis not coinciding with the screw insertion direction. Correspondingly, the tip of the screwdriver will also be conveniently shaped so that it can effectively engage in the screw head at a given tilt angle.

Various types of screws and screwdrivers for different angle ranges are known and available on the market. The individual geometric shapes of the various components used may limit the field of application.

In other cases where the access angle is too wide cement bonding is even preferred to the screwing technique, but this means losing all the advantages offered by the latter.

Document US2016/0151127A1 is known, which describes a special implantology screw having a head with a hemispherical seat suited to house a corresponding hemispherical head of a joining element having, at the opposite end, a seat in which a screwdriver can be engaged.

The spherical joining element can rotate within the screw seat, in such a way as to selectively orient the screwdriver engagement seat and thus allow screwing operations at various tilt angles to be carried out. The drawback of this type of device lies in that it necessarily requires not only a particular type of joining element but also a particular type of screw, that is, it cannot be used with standard implant screws. Document US2019/274793 A1 describes a screwdriver with a specially configured tip in a rounded, multilobed shape and a screw having a correspondingly shaped engagement seat, so that the screwdriver can screw/unscrew the screw even from an angled position. This is due to the rounded shape of the lobes or protrusions of the screwdriver tip, which engage in the corresponding recesses of the screw seat even when the screwdriver is in an inclined position with respect to the screw itself. A drawback of this type of screwdriver lies in that the tip is particularly flattened, in such a way as to make it possible to incline the screwdriver at various angles, even very large angles. Due to said flattened tip, however, only a narrow contact surface with the screw seat can be obtained, which results in localized stress that could damage the screw seat during the screwing/unscrewing operation.

Document US2022/313400A1 describes another type of screwdriver similar to the one described above, in which the tip is trilobed and has a conical apex.

### SUMMARY

The present invention pertains to a screw driver as defined in claims 1 to 6 as well as to sets comprising at least one screwdriver according to one or more of the claims from 1 to 6.

In particular, in order to overcome all the drawbacks mentioned above, a new type of screwdriver for prosthetic screws and a screw that can be manoeuvred by means of said new screwdriver have been designed and manufactured. The present patent also concerns a possible joining element between the screwdriver and the screw, particularly configured to be used with the new screwdriver and with standard screws of any type.

More specifically, the new screwdriver allows prosthetic screws to be screwed even in angled positions, that is, with the screwdriver axis inclined with respect to the axis of the screw.

The characteristics of the new screwdriver are better clarified in the following description, making reference to the drawings, which are attached by way of nonlimiting example.

### BRIEF DESCRIPTION OF THE DRAWINGS

Figure 1 shows a side view of the new screwdriver (100), while Figure 2 shows a detail of the bulb (150) of the screwdriver (100).
Figure 3 shows a sectional view of the bulb (150) drawn along section plane (A-A) shown in Figure 2.
Figure 4 shows the rough and simplified outline of the bulb (150) so that it is possible to observe its overall longitudinal section.

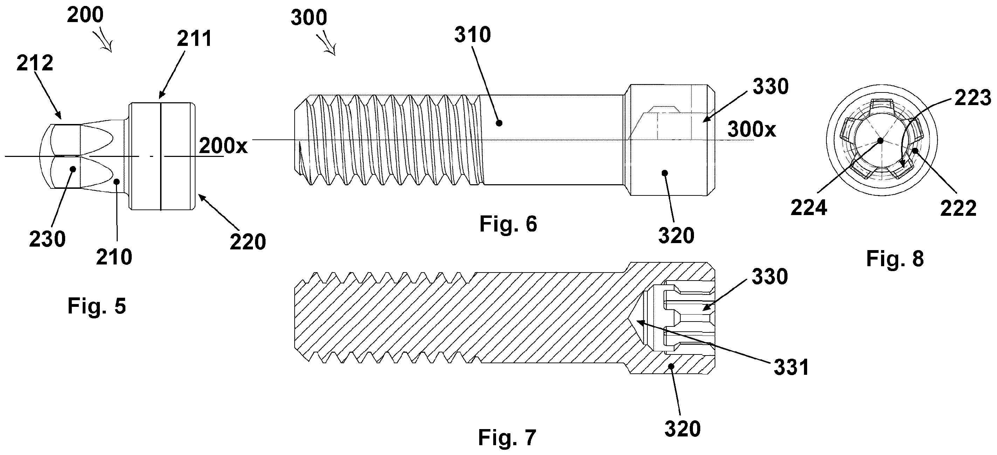

Figure 5 shows a side view of a joining element (200) suited to be used with the new screwdriver (100).
Figure 6 shows a side view of a screw (300) suited to be used with the new screwdriver (100), while Figure 7 shows a longitudinal section of the same.
Figure 7 shows the seat (220) in the joining element (200) of Figure 5, configured so that the bulb (150) of the screwdriver (100) can engage therein. Said seat (220) is identical to the seat (330) of the head (320) of the screw (300) of Figures 6 and 7.
Figures 9 and 10 show how the bulb (150) of the screwdriver (100) engages in the seat (220) in the joining element (200). The same concept can be applied in the case where the joining element (200) is replaced by the screw (300) as shown in Figures 6 and 7.
Figures 11 and 12 schematically show how the new screwdriver (100) is used to screw a standard screw (V) in an implant (I) and fix a post and core (M) by means of said joining element (200).
Figure 13 shows a detail of the recessed edge (225) of the seat (220) in the joining element (200) of Figure 5 and of the seat (330) in the head (320) of the screw (V) of Figure 6.

### DETAILED DESCRIPTION

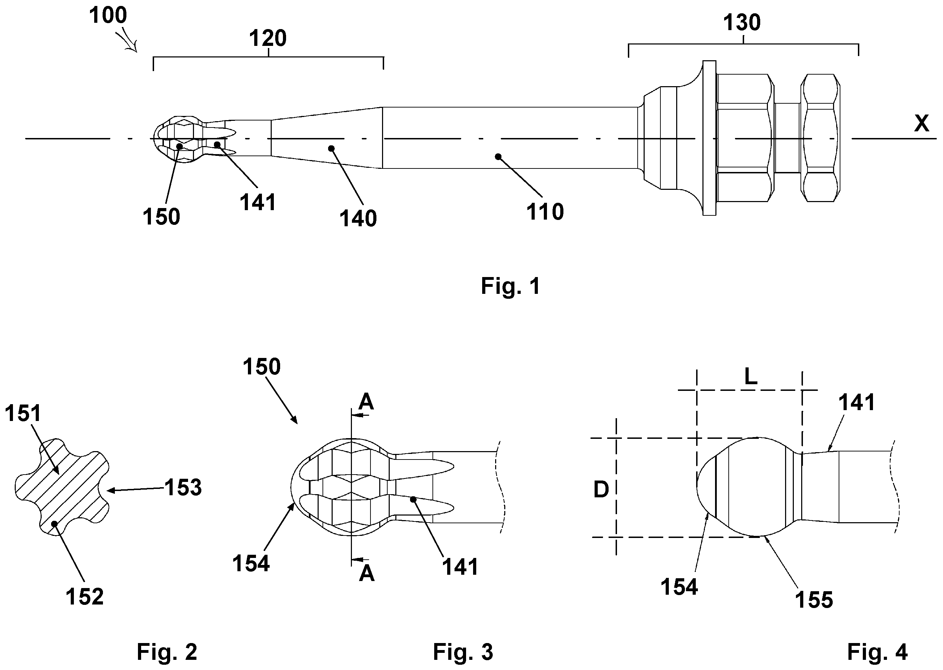

The new screwdriver (100) comprises a shank (110) having a main longitudinal axis (X) and a specially configured tip (120). The screwdriver (100) preferably also comprises a grip for normal manual use or an RA type connecting part for use with a handpiece or a connecting part for digital use or a connecting part (130) for use with a dynamometric ratchet, as shown in Figure 1.

The tip (120) comprises, starting from the shank (110), a connecting part or neck (140) and a terminal part or bulb (150).

Preferably, said neck (140) extends along said longitudinal axis (X) and its diameter decreases progressively towards the bulb (150), being, for example, substantially in the shape of a truncated cone or in any case tapered towards the bulb (150). According to the invention, the neck diameter can vary gradually, resulting in a small angle, or in a discontinuous manner, even with 90° steps.

In the preferred solution, at least one portion (141) of said neck (140), and more specifically the portion (141) connecting it to said bulb (150), has a multilobed cross section, preferably but not necessarily five-lobed, wherein by cross section we mean the cross section drawn on a plane orthogonal to said longitudinal axis (X) of the screwdriver (100).

Said bulb (150) also has a predominantly multilobed cross section, in a manner corresponding to said connecting portion (141), as shown in Figures 2 and 3, that is, it comprises a substantially circular central part (151) from which several protrusions (152), preferably and substantially rounded, extend, wherein said protrusions (152) alternate with as many depressions (153) and are homogeneously distributed around said central part (151),

Said protrusions are preferably 4 or more, and more preferably 5, as it has been observed that less than 4 protrusions would not guarantee a stable engagement of the screwdriver in the corresponding screw or joining element as described below.

The longitudinal section of the bulb (150), that is, the section drawn on a plane containing said axis (X) of the screwdriver (100), has a substantially ogival or ellipsoidal shape, with the major axis coinciding with said longitudinal axis (X), meaning that its side surface can be inscribed in a curved convex surface with an elongated rounded apex (154), as shown in Figure 4, and in any case having a rounded side.

According to the claimed invention, the longitudinal section of the bulb (150) has, starting from said connecting portion (141), a first substantially spherical portion (155) without spherical caps and said elongated apex (154) with rounded sides.

Said elongated ogival/ellipsoidal shape of the bulb (150) mainly serves the function of limiting the maximum angle of the screwdriver with respect to the j oining element (200) or the screw (300), so that no abnormal use of the screwdriver (100) occurs when said angle is exceeded.

For example, in the cases where the screwdriver (100) is used with an excessive inclination, an unwanted disengagement of the screwdriver (100) from the joining element (200) or the screw (300) may occur, or even damage to the bulb (150) or the seat (220, 330) in the joining element (200) or the screw (300).

In particular, the length (L) of the bulb (150) exceeds its maximum diameter (D). For example, its length is included between 1 and 4 mm and its maximum diameter is included between 1 and 4 mm.

The present patent also concerns a joining element (200) suited to be used to transmit the axial rotational motion between said screwdriver (100) and a screw (V) to be tightened.

Said joining element (200) has, for example, a substantially cylindrical body (210) with a seat (220), in which said bulb (150) of the screwdriver (100) can be engaged, at one end (211) and a tip (230) suited to become engaged in a screw (V) of any type at the opposite end (212), for example a standard implant screw.

In particular, said tip (230) of the joining element (200) is configured in such a way that, when the tip (230) is correctly inserted in a screw (V), said joining element (200) is constrained in a position in which it is coaxial with said screw (V), meaning that it cannot be tilted with respect to the latter.

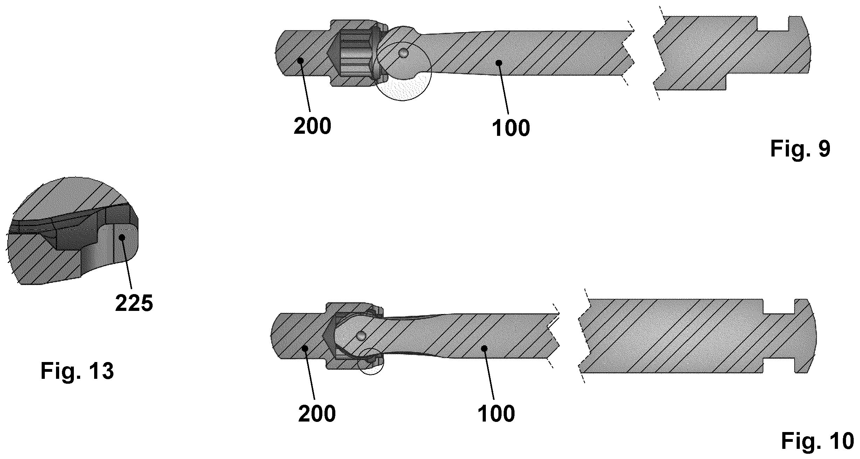

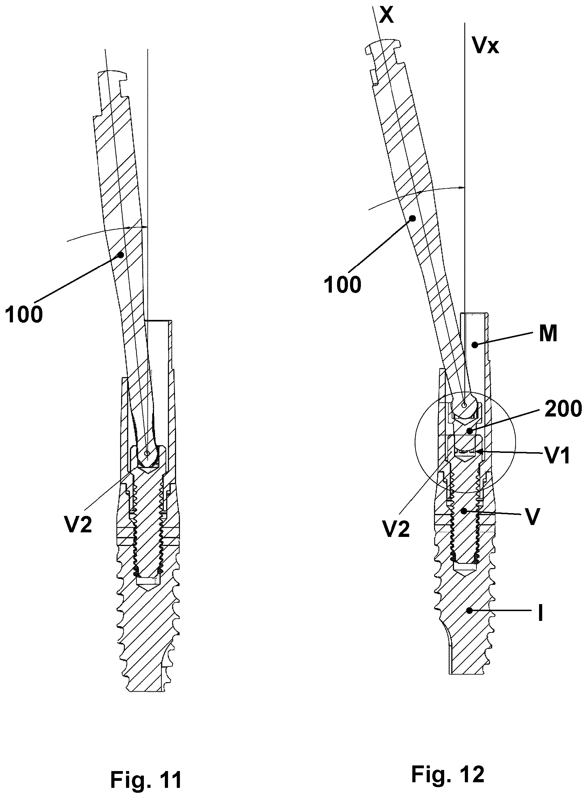

Said joining element (200) makes it possible to screw a standard screw (V) tilting the screwdriver (100) with respect to the axis (Vx) of the screw (V), as shown in Figures 10 and 11.

Furthermore, the presence of said joining element (200) actually lifts the bulb (150) of the screwdriver (100) and thus makes it possible to increase the maximum inclination of the screwdriver in the post and core (M) to be fixed to the implant (I). Said seat (220) in the joining element (200) has a multilobed cross section substantially corresponding to the largest cross section of the bulb (150), and thus comprises a substantially circular central part (221) and several depressions (223) alternating with protrusions (222).

The deepest part (224) of the seat (220), instead, is generically convex and tapered to ensure the controlled mobility of said apex (154) of the bulb (150).

Said tip (230) of the joining element (200), on the other hand, can have any standard shape suitable for engagement with standard prosthetic screws (V).

In order to tighten a screw (V), it will then be sufficient to insert the tip (230) of the joining element (200) in the seat (V1) in the head (V2) of the screw (V) so that the joining element (200) is coaxial with the axis (Vx) of the screw (V) and use the screwdriver (100) on the joining element (200).

The bulb (150) of the screwdriver (100) can engage in the seat (220) in the joining element (200) with inclination generally included between 0 and 40°.

The rotation of the screwdriver (100) around its axis (X) thus causes the joining element (200) to rotate around its axis (X) and consequently the screw (V) to be tightened/loosened.

Figures 9 and 10 show how the bulb (150) of the screwdriver (100) engages in the seat (220) in the joining element (200). Said seat (220) has a recessed edge (225), shown in Figure 13, which ensures that the bulb (150) correctly engages in the joining element (200) and prevents it from accidentally coming out. For this purpose, the diameter of said bulb (150) is larger than that of said connecting portion (141), in order to create a sort of recess in which said recessed edge (225) fits, once the bulb (150) has been correctly inserted in said seat (220) in the joining element (200).

The present patent also concerns a screw (300) configured to be used directly with the new screwdriver (100), that is, without said joining element (200).

The new screw (300) comprises a threaded body (310) and a head (320) provided with a seat (330) configured so that said bulb (150) of the screwdriver (100) can engage therein.

Said seat (330) of the screw (300) is substantially identical to said seat (220), already described above, of said joining element (200). More specifically, it has a multilobed cross section substantially corresponding to the largest cross section of the bulb (150), a deeper part (331) for the apex (154) of the bulb (150) and a recessed anti-slip edge (225), visible in Figure 13.

The bulb (150) of the screwdriver (100) can engage in the seat (330) in the head (320) of the screw (300) with inclination generally included between 0 and 40°.

The rotation of the screwdriver (100) around its axis (X) thus causes the screw (300) to rotate around its axis (300x) and consequently to be tightened/loosened.

Said screw (300) and said joining element (200) can have several possible lengths, in such a way as to shift the height of the bulb (150) of the screwdriver and thus maximise the tilt angle of the screwdriver (100).

This is particularly interesting when working with very long crowns or post and cores (M).

Therefore, with reference to the above description and the attached drawings, the following claims are made.

## Claims

1. Dental implant screwdriver (100), comprising a shank (110) having a main longitudinal axis (X) and a tip (120) comprising, starting from the shank (110), a connecting part or neck (140) and a terminal part or bulb (150), wherein
- said bulb (150) has a predominantly multilobed cross section, that is, it comprises a substantially circular central part (151) from which several protrusions (152), preferably and substantially rounded, extend, wherein said protrusions (152) alternate with as many depressions (153) and are homogeneously distributed around said central part (151),
**characterized in that**
- said bulb (150) has a longitudinal section in a substantially ogival or ellipsoidal compound shape, with the major axis coinciding with said longitudinal axis (X), meaning that it comprises, starting from a portion (141) that connects it to said neck (140), a first substantially spherical portion (155) without spherical caps and an apex (154) in an elongated shape with rounded sides joined to said first portion (155).

2. Screwdriver (100) according to claim 1, **characterized in that** said protrusions are at least 4 and are more preferably 5.

3. Screwdriver (100) according to claim 1, **characterized in that** said neck (140) extends along said longitudinal axis (X) and its diameter progressively decreases, gradually and/or discontinuously, towards said bulb (150).

4. Screwdriver (100) according to claim 1, **characterized in that** at least one portion (141) of said neck (140), and more specifically the portion (141) connecting it to said bulb (150), has a multilobed cross section corresponding to that of said bulb (150).

5. Screwdriver (100) according to the preceding claims, **characterized in that** the diameter of said bulb (150) is larger than that of said connecting portion (141).

6. Screwdriver (100) according to one or more of the preceding claims, **characterized in that** it comprises a grip for normal manual use or an RA type connecting part for use with a handpiece or a connecting part for digital use or a connecting part (130) for use with a dynamometric ratchet.

7. Set comprising at least one screwdriver (100) according to one or more of the preceding claims and at least one joining element (200) for dental implantology, the latter being suited to be used for transmitting the axial rotational motion between said screwdriver (100) and a screw (V) to be tightened, **characterized in that** said joining element (200) has a substantially cylindrical body (210) having a seat (220), in which said bulb (150) of the screwdriver (100) can engage, at one end (211) and a tip (230) suited to engage in a screw (V) at the opposite end (212), in such a way that said joining element (200) is constrained in a coaxial position with respect to said screw (V), and wherein said seat (220) in the joining element (200) has a multilobed cross section substantially corresponding to the largest cross section of the bulb (150), and thus comprises a substantially circular central part (221) and several depressions (223) alternating with protrusions (222).

8. Set according to the preceding claim, **characterized in that** said seat (220) in said joining element (200) is provided with a recessed edge (225) which ensures the correct engagement of the bulb (150) of said screwdriver (100) and prevents it from accidentally coming out.

9. Set comprising at least one screwdriver (100) according to one or more of the claims from 1 to 6 and at least one screw (300) for dental implantology, **characterized in that** said screw (300) comprises a threaded body (310) and a head (320) provided with a seat (330) configured for the engagement of said bulb (150) of said screwdriver (100), and wherein said seat (330) has a multilobed cross section substantially corresponding to the largest cross section of the bulb (150), and therefore comprises a substantially circular central part (221) and multiple depressions (223) alternating with protrusions (222).

10. Set according to the preceding claim, **characterized in that** said seat (330) is provided with a recessed edge (225) that ensures the proper engagement of the bulb (150) of the screwdriver (100) and prevents it from accidentally coming out.

11. Set according to any of the claims from 7 to 10, comprising at least one screwdriver (100) and two or more of said joining elements (200) and/or screws (300), **characterized in that** said two or more joining elements (200) and/or screws (300) have at least two different lengths which are such as to determine different heights of said seats (220, 330) in which said bulb (150) of said screwdriver (100) engages.

## Patentansprüche

1. Dentalimplantat-Schraubendreher (100), umfassend einen Schaft (110) mit einer Hauptlängsachse (X) und einer Spitze (120), umfassend, ausgehend vom Schaft (110), einen Verbindungsteil oder Hals (140) und einen Endteil oder ein Endstück (150), wobei
- das besagte Endstück (150) einen überwiegend mehrlappigen Querschnitt aufweist, das heißt, es umfasst einen im Wesentlichen kreisförmigen Mittelteil (151), von dem sich mehrere Vorsprünge (152), vorzugsweise und im Wesentlichen abgerundet, erstrecken, wobei sich die besagten Vorsprünge (152) mit ebenso vielen Vertiefungen (153) abwechseln und gleichmäßig um den besagten Mittelteil (151) verteilt sind,
**dadurch gekennzeichnet, dass**
- das besagte Endstück (150) einen Längsschnitt in einer im Wesentlichen ogivalen oder ellipsoiden Verbundform aufweist, dessen Hauptachse mit der besagten Längsachse (X) zusammenfällt, was bedeutet, dass es ausgehend von einem Abschnitt (141), der es mit dem besagten Hals (140) verbindet, einen ersten im Wesentlichen kugelförmigen Abschnitt (155) ohne kugelförmige Kappen und einen Scheitel (154) in einer länglichen Form mit abgerundeten Seiten, die an den besagten ersten Abschnitt (155) angeschlossen sind, umfasst.

2. Schraubendreher (100) nach Patentanspruch 1, **dadurch gekennzeichnet, dass** die besagten Vorsprünge mindestens 4 und bevorzugter 5 sind.

3. Schraubendreher (100) nach Patentanspruch 1, **dadurch gekennzeichnet, dass** sich der besagte Hals (140) entlang der besagten Längsachse (X) erstreckt und sein Durchmesser allmählich und/oder diskontinuierlich in Richtung des besagten Endstücks (150) abnimmt.

4. Schraubendreher (100) nach Patentanspruch 1, **dadurch gekennzeichnet, dass** mindestens ein Abschnitt (141) des besagten Halses (140) und insbesondere der Abschnitt (141), der ihn mit dem besagten Endstück (150) verbindet, einen mehrlappigen Querschnitt aufweist, der dem des besagten Endstücks (150) entspricht.

5. Schraubendreher (100) nach den vorhergehenden Patentansprüchen, **dadurch gekennzeichnet, dass** der Durchmesser des besagten Endstücks (150) größer ist als der des besagten Verbindungsabschnitts (141).

6. Schraubendreher (100) nach einem oder mehreren der vorhergehenden Patentansprüche, **dadurch gekennzeichnet, dass** er einen Griff für die normale manuelle Verwendung oder ein Verbindungsteil vom RA (Right Angle)-Typ für die Verwendung mit einem Handstück oder ein Verbindungsteil für die digitale Verwendung oder ein Verbindungsteil (130) für die Verwendung mit einer Drehmomentratsche umfasst.

7. Satz, umfassend mindestens einen Schraubendreher (100) nach einem oder mehreren der vorhergehenden Patentansprüche und mindestens ein Anschlusselement (200) für die Dentalimplantologie, wobei letzteres dazu geeignet ist, zum Übertragen der axialen Drehbewegung zwischen dem besagten Schraubendreher (100) und einer anzuziehenden Schraube (V) verwendet zu werden, **dadurch gekennzeichnet, dass** das besagte Anschlusselement (200) Folgendes aufweist: einen im Wesentlichen zylindrischen Körper (210), der an einem Ende (211) einen Sitz (220) aufweist, in den das besagte Endstück (150) des Schraubendrehers (100) eingreifen kann, und am gegenüberliegenden Ende (212) eine Spitze (230), die dazu geeignet ist, in eine Schraube (V) einzugreifen, sodass das besagte Anschlusselement (200) in einer koaxialen Position in Bezug auf die besagte Schraube (V) gehalten wird, und wobei der besagte Sitz (220) im Anschlusselement (200) einen mehrlappigen Querschnitt aufweist, der im Wesentlichen dem größten Querschnitt des Endstücks (150) entspricht, und daher einen im Wesentlichen kreisförmigen Mittelteil (221) und mehrere Vertiefungen (223), die sich mit Vorsprüngen (222) abwechseln, umfasst.

8. Satz nach dem vorhergehenden Patentanspruch, **dadurch gekennzeichnet, dass** der besagte Sitz (220) im besagten Anschlusselement (200) mit einer vertieften Kante (225) versehen ist, die den korrekten Eingriff des Endstücks (150) des besagten Schraubendrehers (100) gewährleistet und verhindert, dass es versehentlich herausrutscht.

9. Satz, umfassend mindestens einen Schraubendreher (100) nach einem oder mehreren der Patentansprüche 1 bis 6 und mindestens eine Schraube (300) für die Dentalimplantologie, **dadurch gekennzeichnet, dass** die besagte Schraube (300) Folgendes umfasst: einen Gewindekörper (310) und einen Kopf (320), der mit einem Sitz (330) versehen ist, der für den Eingriff des besagten Endstücks (150) des besagten Schraubendrehers (100) konfiguriert ist, und wobei der besagte Sitz (330) einen mehrlappigen Querschnitt aufweist, der im Wesentlichen dem größten Querschnitt des Endstücks (150) entspricht, und daher einen im Wesentlichen kreisförmigen Mittelteil (221) und mehrere Vertiefungen (223), die sich mit Vorsprüngen (222) abwechseln, umfasst.

10. Satz nach dem vorhergehenden Patentanspruch, **dadurch gekennzeichnet, dass** der besagte Sitz (330) mit einer vertieften Kante (225) versehen ist, die den ordnungsgemäßen Eingriff des Endstücks (150) des Schraubendrehers (100) gewährleistet und verhindert, dass es versehentlich herausrutscht.

11. Satz nach jeglichem der Patentansprüche 7 bis 10, umfassend mindestens einen Schraubendreher (100) und zwei oder mehr der besagten Anschlusselemente (200) und/oder Schrauben (300), **dadurch gekennzeichnet, dass** die besagten zwei oder mehr Anschlusselemente (200) und/oder Schrauben (300) mindestens zwei unterschiedliche Längen aufweisen, die derart sind, dass sie unterschiedliche Höhen der besagten Sitze (220, 330) bestimmen, in die das besagte Endstück (150) des besagten Schraubendrehers (100) eingreift.

## Revendications

1. Tournevis pour implant dentaire (100), comprenant une tige (110) présentant un axe longitudinal principal (X) et une pointe (120) comprenant, à partir de la tige (110), une partie de raccordement ou col (140) et une partie terminale ou bulbe (150), où
- ledit bulbe (150) présente une section transversale principalement multilobée, c'est-à-dire qu'il comprend une partie centrale sensiblement circulaire (151) à partir de laquelle s'étendent plusieurs saillies (152), de préférence et sensiblement arrondies, où lesdites saillies (152) alternent avec autant de creux (153) et sont réparties de manière homogène autour de ladite partie centrale (151),
**caractérisé en ce que**
- ledit bulbe (150) présente une section longitudinale de forme composée sensiblement ogivale ou ellipsoïdale, dont l'axe principal coïncide avec ledit axe longitudinal (X), ce qui signifie qu'il comprend, à partir d'une partie (141) qui le relie audit col (140), une première partie sensiblement sphérique (155) sans calottes sphériques et un sommet (154) de forme allongée aux côtés arrondis reliés à ladite première partie (155).

2. Tournevis (100) selon la revendication 1, **caractérisé en ce que** lesdites saillies sont au nombre d'au moins 4 et, de préférence, de 5.

3. Tournevis (100) selon la revendication 1, **caractérisé en ce que** ledit col (140) s'étend le long dudit axe longitudinal (X) et que son diamètre diminue progressivement, de manière graduelle et/ou discontinue, vers ledit bulbe (150).

4. Tournevis (100) selon la revendication 1, **caractérisé en ce qu'**au moins une partie (141) dudit col (140), et plus précisément la partie (141) le reliant audit bulbe (150), présente une section transversale multilobée correspondant à celle dudit bulbe (150).

5. Tournevis (100) selon les revendications précédentes, **caractérisé en ce que** le diamètre dudit bulbe (150) est supérieur à celui de ladite partie de raccordement (141).

6. Tournevis (100) selon l'une ou plusieurs des revendications précédentes, **caractérisé en ce qu'**il comprend une poignée destinée à une utilisation manuelle classique, ou un raccord de type RA [*Right Angle*] destiné à être utilisé avec une pièce à main, ou un raccord destiné à une utilisation digitale, ou un raccord (130) destiné à être utilisé avec un cliquet dynamométrique.

7. Ensemble comprenant au moins un tournevis (100) selon l'une ou plusieurs des revendications précédentes et au moins un élément de raccordement (200) pour l'implantologie dentaire, ce dernier étant adapté pour être utilisé afin de transmettre le mouvement de rotation axial entre ledit tournevis (100) et une vis (V) à serrer, **caractérisé en ce que** ledit élément de raccordement (200) comprend un corps sensiblement cylindrique (210) présentant un logement (220), dans lequel ledit bulbe (150) du tournevis (100) peut s'engager, à une extrémité (211), et une pointe (230) adaptée pour s'engager dans une vis (V) à l'extrémité opposée (212), de telle sorte que ledit élément de raccordement (200) soit contraint dans une position coaxiale par rapport à ladite vis (V), et où ledit logement (220) dans l'élément de raccordement (200) présente une section transversale multilobée correspondant sensiblement à la plus grande section transversale du bulbe (150), et comprend ainsi une partie centrale sensiblement circulaire (221) et plusieurs creux (223) alternant avec des saillies (222).

8. Ensemble selon la revendication précédente, **caractérisé en ce que** ledit logement (220) dans ledit élément de raccordement (200) est muni d'un bord en retrait (225) qui assure l'engagement correct du bulbe (150) dudit tournevis (100) et empêche qu'il puisse sortir accidentellement.

9. Ensemble comprenant au moins un tournevis (100) selon l'une ou plusieurs des revendications 1 à 6 et au moins une vis (300) pour l'implantologie dentaire, **caractérisé en ce que** ladite vis (300) comprend un corps fileté (310) et une tête (320) pourvue d'un logement (330) configuré pour l'engagement dudit bulbe (150) dudit tournevis (100), et où ledit logement (330) présente une section transversale multilobée correspondant sensiblement à la plus grande section transversale du bulbe (150), et comprend donc une partie centrale sensiblement circulaire (221) et des creux multiples (223) alternant avec des saillies (222).

10. Ensemble selon la revendication précédente, **caractérisé en ce que** ledit logement (330) est pourvu d'un bord en retrait (225) qui assure l'engagement correct du bulbe (150) du tournevis (100) et empêche qu'il puisse sortir accidentellement.

11. Ensemble selon l'une quelconque des revendications 7 à 10, comprenant au moins un tournevis (100) et deux ou plusieurs desdits éléments de raccordement (200) et/ou vis (300), **caractérisé en ce que** lesdits deux ou plusieurs éléments de raccordement (200) et/ou vis (300) ont au moins deux longueurs différentes qui sont telles qu'elles déterminent des hauteurs différentes desdits logements (220, 330) dans lesquels s'engage ledit bulbe (150) dudit tournevis (100).
